# EUROPEAN PATENT SPECIFICATION

(11) **EP 1 107 773 B1**
(45) Date of publication and mention of the grant of the patent: **03.01.2007**
(21) Application number: 99943941.7
(22) Date of filing: 25.08.1999
(51) Int. Cl.: G01N 33/569, C07K 16/12

(54) **METHOD FOR DETECTION OF LEGIONELLA BACTERIA EMPLOYING PURIFIED ANTIGEN-SPECIFIC ANTIBODIES**
VERFAHREN ZUR DETEKTION VON LEGIONELLA BAKTERIEN UNTER VERWENDUNG AUFGEREINIGTER ANTIGEN-SPEZIFISCHER ANTIKÖRPER
TECHNIQUE DE DETECTION DE BACTERIES LEGIONELLA UTILISANT DES ANTICORPS PURIFIES SPECIFIQUES D'ANTIGENE

(30) Priority: 25.08.1998 US 139720
(43) Date of publication of application: 20.06.2001
(73) Proprietor: Binax, Inc., Portland, ME 04103 (US)
(72) Inventor: MOORE, Norman, James, North Berwick, ME 03906 (US); MOLOKOVA, Elena, Valentin, Portland, ME04101 (US); KOULCHIN, Vladimir, Andrei, Portland, ME 04101 (US)
(74) Representative: Becker Kurig Straus
(86) International application number: PCT/US1999/019506
(87) International publication number: WO 2000/010584

(56) References cited:
- US-A- 4 206 094
- US-A- 4 411 832
- US-A- 4 780 407
- US-A- 5 415 994
- MOORE N ET AL: "DEVELOPMENT OF AN IMMUNOCHROMATOGRAPHIC (ICT) ASSAY FOR THE DETECTION OF LEGIONELLA PNEUMOPHILA" ABSTRACTS OF THE GENERAL MEETING OF THE AMERICAN SOCIETY FOR MICROBIOLOGY, THE SOCIETY, WASHINGTON, DC, US, vol. 98, May 1998 (1998-05), page 203, XP009024507 ISSN: 1060-2011
- JUERGENS D ET AL: "CROSS-REACTING LIPOPOLYSACCHARIDE ANTIGENS IN LEGIONELLA PNEUMOPHILA SEROGROUPS 1 TO 14" INFECTION AND IMMUNITY, AMERICAN SOCIETY FOR MICROBIOLOGY. WASHINGTON, US, vol. 63, no. 6, June 1995 (1995-06), pages 2180-2184, XP002925153 ISSN: 0019-9567
- KNIREL ET AL.: 'The Structure of the O-Specific Chain of Legionella pneumophila Serogroup I Lipopolysaccharide' EUR. J. BIOCHEM. vol. 221, 1994, pages 239 - 245, XP002925152
- JURGENS ET AL.: 'Cross-Reacting Lipopolysaccharide Antigens in Legionella pneumohpila Serogroups 1 to 14' INFECTION AND IMMUNITY vol. 63, no. 6, June 1995, pages 2180 - 2184, XP002925153
- BARTHE ET AL.: 'Common Epitope on the Lipopoylsaccharide of Legionella pneumophila Recognized by a Monoclonal Antibody' JOURNAL OF CLINICAL MICROBIOLOGY vol. 26, no. 5, May 1988, pages 1016 - 1023, XP002925154

## Description

This invention relates to protein-free carbohydrate, including polysaccharide antigens separated from bacteria of the genus Legionella, and especially from serogroups and/or strains of Legionella pneumophila, including the O-polysaccharide antigen of L. pneumophila serogroup 1, and to the use of these antigens in the affinity purification of polyvalent antibodies to corresponding Legionella organisms. More particularly, the invention encompasses coupling the carbohydrate or polysaccharide antigen separated from a Legionella bacterium to an activated chromatographic column and using that column for affinity purification of the polyclonal antibodies to the same species, or the same serogroup of a species, of Legionella. The invention further encompasses the use of the affinity-purified polyvalent antibodies produced in immunochemical assays for the detection of Legionella-caused diseases such as Legionnaires disease and Pontiac fever in human patients and for the detection of environmental sources of Legionella infectious agents.

### BACKGROUND

Legionnaires' disease, a pneumonia-like human infection caused by gram-negative bacteria of the genus Legionella, is virtually impossible to differentiate on a reliable clinical basis (involving assessment of patient symptoms without laboratory tests) from pneumonia and other similar lung infections. Because the disease may produce lung abscesses, infections in other bodily organs or bacteremia, and its mortality rate is significantly increased by delay in commencing appropriate therapy, there is a need for rapid and reliable diagnostic tests which has to date been only partially met. Stout, J.E. and Yu, V.L., "Legionellosis", 337, N. Eng. J. Med. 682-687 (1997). Efforts to develop such tests have been hampered by the fact that there are a number of Legionella species, at least some of which are known to have a number of distinct serogroups.

Legionnaires' disease was first recognized in the summer of 1976 and a-number of techniques for detecting Legionella ("L.") pneumophila, which is now known to account for some 90% of cases (Stout and Yu, supra), have been developed in the interim. In general, these tests are time-consuming and incapable of identifying more than one serogroup of the 15 serogroups known so far to fall within the L. pneumophila species. It should be noted, however, that, as reported by Stout and Yu, supra, more than 80 % of reported cases of Legionnaires' disease are attributable to L. pneumophila serogroup 1 -- a fact which makes the development of a rapid, reliable immunoassay for that entity of particular importance and has led researchers to focus on this as a priority.

The early efforts to establish the identity of the causative agent of Legionnaires' disease depended largely upon culturing the bacteria for 5 to 6 days and examining the culture microscopically. Efforts to speed up the identification process led to numerous immunochemical tests of varying sensitivity and specificity including, inter alia, the presently commercially available EQUATE^{™} radioimmunoassay ("RIA"), and the Binax enzyme immunoassay ("EIA"), both of which are sold in kit form by applicants' assignee, Binax, Inc. As indicated by Hackman, B.A. et al. in "Comparison of Binax Legionella Urinary Antigen EIA Kit with Binax RIA Urinary Antigen Kit for Detection of Legionella pneumophila Serogroup I Antigen", 34 J. Clin. Microbiol. 1579-1580 (1996), both of these assays were found to be specific for L. pneumophila Serogroup I antigen. The reported sensitivity of the EIA was 77%; that of the RIA was higher. The article indicates that each can be performed within "less than 3 h[ours] from beginning to end"; in Binax's own tests each requires at least 2 1/2 hours to perform. More information about this EIA assay appears in Kazandjian, D. et al., "Rapid Diagnosis of Legionella pneumophila Serogroup I Infection with Binax Enzyme Immunoassay Urinary Antigen Test", 35 J. Clin Immunobiol. 954-956 (1997).

In Abstracts of the general meeting of the American society for microbiology, The Society, Washington DC, US, Vol. 98, may 1998 (1998-05), p. 203, Moore N. et al. reported about the "development of an immunochromatographic (ICT) assay for the detection of Legionella pneumophila" by Binax, Inc. The assay has been developed in lateral flow membrane format (ICT) and is directed to the screening of Legionella pneumophila serogroup 1. Carbohydrate antigen of 95% purity was isolated and coupled to a Sepharose 4B matrix. Rabbit antibodies were raised, affinity purified, immobilized and conjugated to colloidal gold to obtain an ICT assay.

### BRIEF DESCRIPTION OF THE INVENTION

Perhaps the most significant advantage of the immunochromatographic test ("ICT") described herein is its ability to give a test result within a 15-minute time span for the presence or absence of L. pneumophila Serogroup I (or its antigen), which result is of high specificity and sensitivity. The speed with which this test can be reliably conducted to yield a result of high specificity and sensitivity is believed to be due to the strongly reactive nature of the affinity purified antibodies prepared in accordance with this invention. The superior reactive properties of these antibodies, in turn, is believed to be attributable to the use for affinity purification of the novel, essentially protein-free O-polysaccharide antigen of L. pneumophila serogroup 1 which is also a part of this invention.

Another ICT test has been made possible by the separation, according to this invention, of an antigen of L. pneumophila serogroup 5 which is of non-proteinaceous, carbohydrate nature and is common to multiple serogroups of L. pneumophila. The presence of such a common antigen has been suggested in the scientific literature; see e.g., Nolte, F.S. et al., "Electrophoretic and Serological Characterization of the Lipopolysaccharides of Legionella pneumophila", 52 Infection and Immunity 676-681 (1986); Otten, S. et al., "Serospecific Antigens of Legionella pneumophila", 167 J. Bacteriol. 893-904 (1986); Barthe, C. et al., "Common Epitope on the Lipopolysaccharide of Legionella pneumophila Recognized by a Monoclonal Antibody", 26 J. Clin. Microbiol. 1016-1023 (1988); Knirel, Y.A. et al., "The Structure of the O-specific Chain of Legionella pneumophila Serogroup I", 227 Eur. J. Biochem. 239-245 (1994). Heretofore there has been no clear report of any separation of such an antigen for use in developing a useful broad spectrum immuno-chemical assay.

This invention comprises the extraction from any Legionella bacterium, and especially from a bacterium of any of the serogroups of L. pneumophila of a protein-free carbohydrate antigen, the preparation of a conjugate of this antigen with a spacer protein, the coupling of the conjugate to an affinity column, the use of that column for the purification of polyvalent antibodies to the corresponding Legionella bacterium, such as a bacterium of a serogroup of L. pneumophila and the use of the antibodies thus purified in an ICT immunoassay for detecting Legionella bacteria or their antigens, including antigens of L. pneumophila serogroups or their corresponding bacteria, as more specifically described hereinafter.

In particular, the invention includes the separation of a protein-free O-polysaccharide antigen specific to L. pneumophila serogroup 1, its use in the affinity purification of the polyvalent antibody specific to the same microorganism and the use of that affinity purified antibody in an ICT immuno-assay of high specificity and high sensitivity that is performable within 15 minutes.

In another embodiment, the invention includes the separation from L. pneumophila serogroup 5 of a protein-free carbohydrate antigen, and its use in the affinity purification of the polyclonal antibody specific to the L. pneumophila serogroup 5 antigen (i.e., an antibody that was obtained from a rabbit immunized with the said antigen); this antibody showed an ability to cross-react with antigens of the L. pneumophila serotypes 1, 2 and 4 in addition to the antigen of serotype 5.

### DESCRIPTION OF THE DRAWINGS

Figure 1 and its related Figures 1A, 1B and 1C hereof show the structure of a typical ICT device which is suitably adapted to perform the L. pneumophila serogroup 1 specific assay, as described in Examples VII, VIII and IX hereof.
Figure 2 hereof shows the results of Western blot analyses of phosphate-buffered saline extracts of L. pneumophila serogroups 1, 2, 4 and 5 with L. pneumophila serogroup 5, protein-free carbohydrate antigen-affinity-purified polyclonal antibody specific to serogroup 5 antigen, which analysis is described in Example X hereof.

### DETAILED DESCRIPTION OF THE INVENTION

Previous experience in the art, including experience with the Binax RIA assay for L. pneumophila Serogroup 1 antigen sold under the trademark EQUATE and the Binax EIA assay for the same antigen, has shown that antigens of the Legionella species, including antigens of various L. pneumophila serogroups, are-more conveniently detectable in specimens from the urine of patients infected with a Legionella microorganism than in specimens of blood, sputum or other fluids. This is in part because the Legionella antigens usually appear in urine within 1-3 days after infection of a human patient whereas their appearance at a detectable level in blood may occur later, and also in part because patients infected with Legionnaires' disease often do not produce much sputum. The ICT test which forms a part of this invention can be configured to run on blood, sputum or some other fluid such as cerebrospinal fluid, or on aqueous samples of environmental origin. It is noted that urine is generally the preferred sample fluid for diagnosis of human patients because it can be obtained non-invasively and easily, even in the doctor's office, and it is not as readily contaminated with other microorganisms, e.g., oral microflora present in sputum, which are innocuous but may affect the results obtained.

Broadly speaking, the ICT test for L. pneumophila serogroup 1 antigen that is specifically a part of the present invention may be designed to be run in any known disposable ICT device disclosed in the art. Preferably, the test is conducted using an ICT device of the type disclosed in copending U.S. Application Serial. No. 07/706,639 of Howard Chandler, or one of its continuation-in-part applications, all assigned to Smith-Kline Diagnostics, Inc. but exclusively licensed to Binax, Inc. in a wide area of applications that includes the present diagnostic field - i.e., diseases of the respiratory system. The device is suitably impregnated with the affinity purified polyvalent antibodies herein disclosed which are specific to the antigen of L. pneumophila serogroup 1 antigen. Positive results of the assay are shown by the appearance of a color upon reaction of suitably labeled antibodies with the antigen. Suitable labels may be any of those known in the art to produce visible color when antibodies conjugated thereto react with antigen, including finely divided metallics and various other labeling materials. Colloidal gold is the preferred label.

The invention contemplates that protein free carbohydrate or antigens may similarly be separated from each of the Legionella-bacteria, including from bacteria of other Legionella species and from bacteria of other serogroups of L. pneumophila, that such antigens may similarly be utilized in the affinity purification of polyvalent antibodies to the corresponding Legionella species or serogroup bacteria and its antigen, and that these affinity-purified antibodies may be utilized in ICT and other immunoassays as specifically disclosed herein for affinity-purified antibodies to L. pneumophila serogroup 1.

Preliminary to the preparation of the device is obtaining the antibodies and effecting their affinity purification. The antibodies that may be used in this invention are conventional polyvalent (also called "polyclonal") antibodies obtained by the well known process of immunizing a rabbit, goat or other animal to a Legionella antigen, e.g., L. pneumophila antigen of known serogroup and bleeding the animal after the passage of an appropriate time period to obtain serum containing the desired antibodies. See, e.g., Cherry, U.B. and McKinney, R.M., pp. 91-104 in Jones, G.L. and Herbert, G.A. (Eds.), "Legionnaires'" the disease. the bacterium and the methodology, (Center for Disease Control, Atlanta, 1979). In this invention, relative to the ICT immunoassay for L. pneumophila serogroup 1 disclosed herein, the rabbit or other animal is immunized to L. pneumophila serogroup 1 antigen and the polyvalent antibodies recovered from its blood are antibodies to L. pneumophila serogroup 1.

The polyvalent antibodies to be used in this invention are further subjected to affinity purification on a specially prepared chromatographic column which employs the protein-free carbohydrate (including polysaccharide) antigen of the same bacterium against which the antibodies are reactive as the purifying agent for them.

Preliminary to the affinity purification of the antibodies, it is therefore necessary according to this invention to prepare protein free purified carbohydrate antigen from a culture of known Legionella bacteria of the desired species or serogroup of a species.

The following examples I and II explain how the purified protein free carbohydrate antigen is obtained.

### Example I

Bacteria of L. pneumophila Serogroup 1 (strain Philadelphia-1) were obtained from Centers for Disease Control and Prevention (Atlanta, GA) and cultured on charcoal-yeast extract agar plates obtained from Northeast Laboratory (Waterville, ME) for a period of 72 hours at 37°C. Cells were harvested with phosphate-buffered saline ("PBS") at pH 7.2 containing 0.2% of NaN₃ and collected by centrifugation at 8000 rpm for 25 minutes. The resulting cell pellet was stored at -20°C. until used.

Wet cells from this pellet were suspended in 20 ml. 0.1 M NaOH per gram of wet cells and stirred at room temperature for 45 minutes. The pH of the solution was then adjusted with concentrated acetic acid to 3.0 and the solution was subjected to centrifugation at 8000 rpm for 20 minutes. The supernatant from this step was then neutralized with aqueous NaOH and dialyzed against distilled water.

The resulting dialyzate was concentrated 10 times on a rotary vacuum evaporator and then sonicated for 5 minutes in an ultrasonic bath.

Proteinase K, in a concentration of 0.2 mg. per ml. of the concentrated product, was added to digest the remaining proteins and the mixture was incubated at 40°C. overnight. The next step was the addition of further Proteinase K, in a concentration of 0.1 mg. per ml., to the mixture, followed by further overnight incubation at 40°C. This second incubation was followed by concentration of the product on a rotary evaporator to a small volume, adjustment of its pH to 10-11 with 0.2% triethylamine and application of the thus-treated mixture to a column of Sephacryl S-200 from Pharmacia, equilibrated with 0.02% triethylamine. Material eluted in the first peak was pooled, adjusted with 0.1 NHCl to approximately neutral pH and dialyzed against distilled water for 18 hours, followed by lyophilization.

The yield of O-polysaccharide antigen from 16.5 grams of wet cells of L. pneumophila serogroup 1 strain Philadelphia-1 was 62 mg.

It should be noted that in place of Proteinase K, any broad spectrum protease enzyme preparation may be used. What is important in this procedure is the elimination of protein from the antigen to the maximum possible extent within reasonable limits of time feasibility.

### Example II

Example I was repeated using L. pneumophila serogroup 5 strain Dallas IE as the bacteria in the culture step. 11.5 wet grams yielded 21 mg. of a carbohydrate antigen. At a later time, the procedure was again repeated, this time using L. pneumophila serogroup 5 (strain U8W) as the bacteria in the culture step.

It is within the scope of this invention to repeat the culturing and extraction steps as herein described on any other Legionella bacterium and especially on bacteria of any other of the serogroups of L. pneumophila, i.e., on any of serogroups 2, 3, 4 and 6-15 inclusive, to obtain a carbohydrate antigen free of proteinaceous material.

In order to couple the protein free O-polysaccharide product of Example I or the carbohydrate of Example II to a chromatographic column for use in affinity purification of polyvalent antibodies to the corresponding bacterium from which the carbohydrate antigen was extracted, it is necessary to complex it with a protein spacer molecule suitably prepared to ensure that it will stably bond to both the carbohydrate antigen and the column and will itself remain inert during the affinity purification step. A modified bovine serum albumin ("BSA") was chosen as the preferred spacer molecule and was prepared as in Example III:

### Example III -- Preparation of Protein Spacer Molecule

An 0.5M aqueous solution of hydrazine dihydrochloride from Aldrich Chemical Co., Inc. (Milwaukee, WI) was prepared and its pH was adjusted to 5.2 with dry NaOH. The solution was then mixed with dry BSA from Sigma Chemical Co. to a final concentration of 25 mg. per ml. of BSA. After the BSA was completely dissolved, N-(dimethylaminopropyl)-N¹-ethylcarbodiimide hydrochloride obtained from Fluka Chemical Co. (St. Louis, MO) was added to a final concentration of 2.5 mg. per ml. The mixture was stirred overnight at ambient temperature and then dialyzed for a period of about five days against distilled water at 4°C, with daily changes of the water.

In lieu of this modified BSA, it is contemplated that other appropriate spacer molecules may be used.

The conjugation of the O-polysaccharide antigen from serogroup 1 of L. pneumophila to the spacer molecule was conducted as follows:

### Example IV -- Conjugation of O-polysaccharide Antigen to Spacer Molecules

L. pneumophila serogroup 1 protein-free O-polysaccharide antigen was dissolved in distilled water in a concentration of 4-5 mg/ml and the pH was adjusted to 5.0 with 1 M HCl. The modified BSA solution prepared as in Example III was adjusted to pH 5.0 with 1 M HCl and slowly added, in a weight ratio of 4-to-1 of the O-polysaccharide antigen solution, to the latter. After 5 minutes of stirring, about 100-200 mcl of distilled water containing the N-(dimethylamino-propyl-N¹-ethylcarbodiimide hydrochloride referred to in Example III was added in a weight ratio of 1:2 to the O-polysaccharide antigen/BSA solution. The resulting mixture was stirred at ambient temperature overnight.

To separate the conjugate of the O-polysaccharide antigen with modified BSA from any unreacted materials present, the reaction mixture was chromatographed on a Sepharose CL-4B column equilibrated with a buffer of 0.1 M NaH₂PO₄:0.5 M NaCl. All of the chromatography fractions were subjected to testing for antigen activity using the commercial Binax EIA test referred to above. All fractions that showed antigen activity in the tested were pooled in and used for affinity column preparation as shown in Example V.

### Example V -- Coupling of Conjugate to Activated Chromatographic Column

An immunoadsorbent gel was prepared by conventionally activating Sepharose CL-4B with cyanogen bromide. The ligand of O-polysaccharide antigen with modified BSA, prepared as in Example IV, was coupled to it using procedures known in the art, e.g., as described in Hermanson, G.T., et al. in Immobilized Affinity Ligand Techniques, 53-56 (Academic Press, Inc. 1992). The gel was then packed in a column and washed successively with 5-10 volumes per volume of gel with PBS of pH 7.2, triple strength PBS of pH 7.2 and 0.2 M glycine-HCl of pH 2.5. The resulting activated column was used, as described below, for affinity purification of polyvalent antibodies to L. pneumophila serogroup 1 antigen. The column after elution should be stored in PBS or anther neutral buffer until used.

Instead of cyanogen-bromide activated Sepharose, spherelose and various commercially available activated columns could be used in this step.

### Example VI -- Affinity Purification of Antibodies

The activated column described in Example V was used for the affinity chromatography of rabbit-anti-L. pneumophila serogroup 1 polyvalent antibodies to L. pneumophila serotype 1 antigen according to the method described by Harlow E. and Lane, D. in Antibodies: A Laboratory Manual at 313-315 (Cold Spring Harbor Laboratory, 1988). Elution of the antibodies from the column was effected with 0.2M glycine-HCL buffer of pH 2.5. Alternative eluants such as 3M NaSCN or 0.1M Et₃N could be substituted.

These affinity-purified antibodies were utilized in an ICT test specific to L. pneumophila serogroup 1 as described in the following example.

### Example VII -- ICT Device and Its Preparation

### A. Preparation of Test Device:

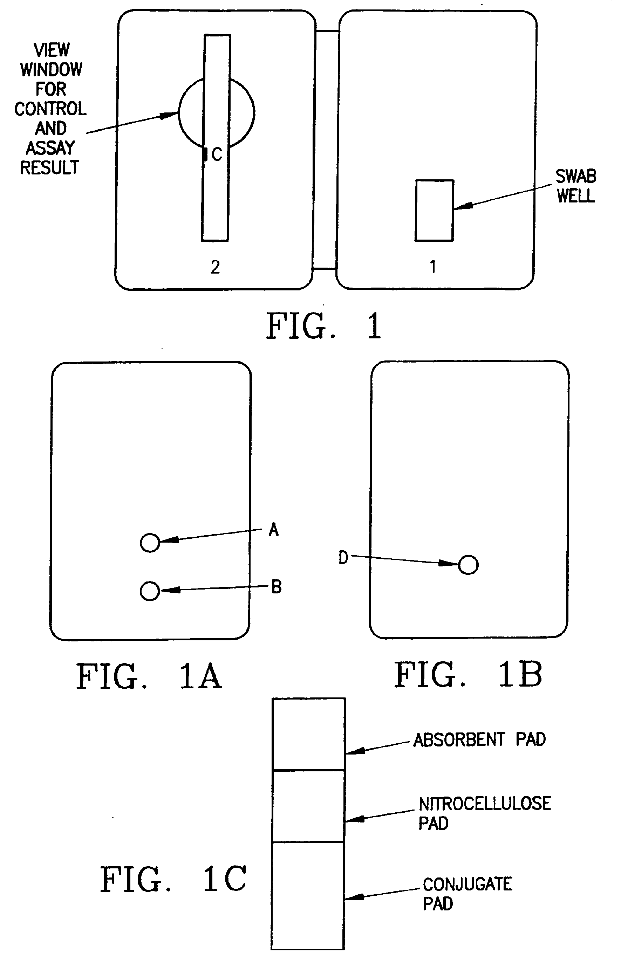

A test device comprising a hinged cardboard housing equipped with a window to allow the viewing of both the test results and control results was prepared as shown in Figure 1. The device has a recess into which is placed a preformed plastic swab well for receiving the sample-wetted swab on the right-hand (labeled 1 in the drawing). An overlabel shown in Figure 1A is then placed over the entire right-hand side of the device. The overlabel has been equipped with two holes -- a lower one (marked B on Figure 1A) into which the saturated swab is to be inserted and an upper one (marked B on Figure 1A) toward which the swab will be pushed after insertion thereof into the hole B. The position of the overlabel with its holes A and B, and the swab well cooperate to hold the swab in a proper position during the assay and to promote the expulsion of sorbed liquid from the swab.

A preassembled test strip (marked C on Figure 1) described below, is inserted into the recess (labeled 2 on Figure 1) and held in place by an adhesive applied to the bottom thereof. An overlabel shown in Figure 1B is placed atop the left-hand side. It has been equipped with a single hole (marked D in Figure 1B) which mates to the right-hand side hole A when the device is closed for performance of the assay.

The assembled device is stored in a sealed pouch with desiccant until it is used. Prior to sealing the pouch and storing, a lightly adhesive tape is placed on the outer edge of the right-hand half of the device.

### B. Construction and Preparation of the Preassembled Test Strip

Figure 1C shows the construction of the preassembled strip. It is comprised of a conjugate pad of sorbent material in which a conjugate of gold particles and the affinity-purified rabbit anti-Legionella pneumophila serogroup 1 antibodies described above have been impregnated. In contact with this pad is a nitrocellulose pad onto which a capture line for the sample which reacts with the conjugate has been established by embedding a stripe of affinity-purified rabbit anti-L. pneumophila serogroup 1 antibodies, prepared as described above. The nitrocellulose pad also has a downstream control line established by striping the pad with goat anti-rabbit immunoglobulin (IgG). Following the nitrocellulose pad, the strip is ended by an absorbent pad which serves as a reservoir for liquid. All of these pads are backed by an adhesive strip when the device is ready to ship.

The conjugate pad is normally made from non-woven polyester or extruded cellulose acetate. To prepare this pad for use in the assay, gold particles of 50 nm. diameter are conjugated to affinity-purified rabbit anti-Legionella pneumophila serotype 1 antibodies prepared as described above. The conjugation is effected using a known method such as that described by DeMay in Polak, J.M. and Van Norden, S. (Eds.), Immunochemistry: Modern Methods and Application, (Wright, Bristol, England, 1986). The gold conjugate particles are mixed with a drying agent consisting of aqueous 5mM sodium tetraborate of pH 8.0 containing 1.0% BSA, 0.1% Triton X-100, 2.0% Tween 20, 6.0% sucrose and 0.02% sodium azide. The pad is heated sufficiently to remove all of the liquid present and stored in a low-humidity environment pending assembly of the test strip. These pads and their treatment are especially chosen so that the pads will hold the dry conjugate and will release it only when later wetted by sample.

The nitrocellulose pad is first treated by embedding a stripe of affinity purified rabbit anti-L. pneumophila serotype 1 antibodies in a first portion thereof, using a carrier solution of phosphate buffered saline. These antibodies act as the capture line. In a second portion of the pad downstream of the first one in the assembled test device, the control line is established by striping goat anti-rabbit IgG in the same carrier solution on the surface of the pad. The nitrocellulose pad is then subjected to desiccation at 18-25° C to promote permanent absorption of the protein stripes thereto.

The absorbent pad used is of a commercially available cellulosic material sold under the name Ahlstrom 939. This pad requires no special treatment.

### C. Kit Preparation

As sold in commerce, the test device containing the finished test strip is assembled. In practice, a number of devices are packaged with a commensurate number of swabs fashioned from fibrous Dacron and a bottle of "Reagent A" equipped with a top adapted to deliver Reagent A dropwise. "Reagent A" is a solution of 2.0% Tween 20, 0.05% sodium azide and 0.5% sodium dodecyl sulfate in a 0.05 M sodium citrate-sodium phosphate buffer of pH 6.5. Positive and negative controls are also included in each kit.

The use of the finished test devices to identify L. pneumophila serogroup 1 O-polysaccharide antigen is illustrated in the following example VIII:

### Example VIII - Conducting the ICT Test for L. pneumophila Serogroup 1 O-Polysaccharide Antigen

In practice, the swab furnished with each device is dipped into the liquid sample, completely immersing the swab head. The use of the swab to act as a filter for undissolved solids, semisolids and colloids present in liquid biological samples such as urine, blood, lymph, etc. and also in liquid environmental samples is the subject of copending Application Serial No. 09/044,677 of Norman Moore and Vincent Sy filed March 19, 1998, which will shortly be assigned to Binax, Inc. The swab is inserted into the hole at the bottom of the device (hole B of Figure 1A) and gently pushed upward so that the swab tip is visible in the top hole (hole A of Figure 1A). The Reagent A vial is held vertically above hole B and two drops of Reagent A are slowly added. The adhesive liner is then immediately peeled from the right edge of the device and the device is closed and securely sealed, thus pressing the swab in the swab well against the gold conjugate pad. After 15 minutes, the result can be read in the window of the device. A negative sample - i.e., one containing no L. pneumophila serogroup 1 O-polysaccharide antigen -- will exhibit only the control line in the top half of the window. A positive sample containing the target antigen will show two lines, the lower one of which is the patient (or sample) line; even a faint sample line indicates the presence of the target antigen in the sample. If no line appears in the window after 15 minutes, or only a sample line appears in the lower part of the window, the test is invalid and must be repeated.

Using the procedure described above, the devices prepared as described in Example VII were tested in the ICT procedure just described against 300 patient urine samples, 100 of which had been previously diagnosed as having L. pneumophila serogroup 1 infection.

These ICT tests according to this invention were conducted under circumstances such that the previous diagnoses were unknown to personnel performing the ICT tests. Overall, 98% of the ICT tests agreed with the previous positive diagnoses. Also overall, 98% of the urine samples previously diagnosed as negative for L. pneumophila serogroup 1 O-polysaccharide antigen gave results in agreement therewith when tested by the ICT procedure described herein, using the ICT device described in Example VII.

### Example IX - Use of the ICT to Test Environmental Samples

Applicability of this same test to environmental samples suspected of containing L. pneumophila serogroup 1 was also investigated as follows:

Water was seeded with L. pneumophila serogroup 1 bacteria obtained from a commercial source. The mixture was concentrated by filtering through a 0.22 µm filter. A swab dipped in the sample was applied to the device, the device was closed and the assay was allowed to proceed. A positive result was observed within less than 15 minutes.

### Example X - Western Blot Immunoassay For Detection of Cross-Reactive Carbohydrate Antigens of L. Pneumophila Serogroups 1, 2, 4 and 5

In order to perform the Western Blot immunoassay using a kit purchased from Bio-Rad Laboratories, L. pneumophila serogroup 5 cells were cultured as in Example II. A suspension of these cells was solubilized with 1% sodium dodecylsulfate in the presence of 10 mM mercaptoethanol at 100° C. for 5 minutes. The solubilized cells were treated with protease K and then subjected to electrophoretic separation of protein according to standard procedures provided by Bio-Rad.

The carbohydrate antigen from L. pneumophila serogroup 5 was conjugated to the spacer molecule described in Example III hereof in the manner described in Example IV and applied to an activated Sepharose column as described in Example V. This column was then used for the affinity purification of polyvalent rabbit antibodies specific to the carbohydrate antigen of L. pneumophila serogroup 5 (which were conventionally obtained from serum of a rabbit previously injected with the protein-containing of L. pneumophila serogroup 5) using the procedure of Example VI.

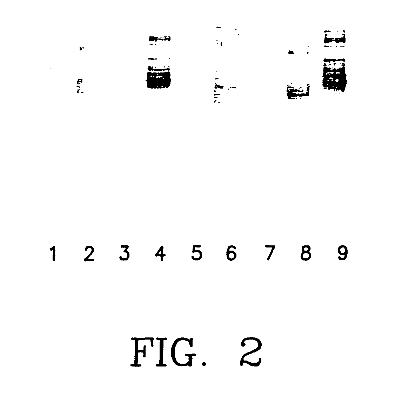

The Western immunoblot analysis was performed using a reagent kit from Bio-Rad and according to directions from this manufacturer. Briefly, the PBS extract of cells of L. pneumophila antigens 1, 2, 4 and 5 was subjected to the SDS-PAGE in 12 % polyacrylamide gel blocked with 1% BSA with PBS transferred onto a nitrocellulose membrane. After this step, the membrane was incubated with affinity purified antibodies specific to carbohydrate of L. pneumophila serogroup 5. The membrane, washed as recommended by the manufacturer, and incubated with horseradish peroxidase conjugated to goat-anti-rabbit antibodies provided by Bio-Rad. After washing, the membrane was developed with a substrate system of 0.022 M 4-chloro-1 naphthol and 0.0012 M N, N-dimethyl-p-phenylene-diamine monohydrochloride in 0.1 M sodium citrate buffer of pH 6.9 containing 2.9 mM of hydrogen peroxide. Figure 2 hereof shows the Western blot assay results compared with that of the prestained SDS-PAGE standard (in Lane 5) for the affinity purified antibodies of serogroup 5 of L. pneumophila against PBS extracts containing antigens of L. pneumophila as follows:
Lanes 1 and 7--L. pneumophila serogroup 2 (strain Togus-1)
Lanes 2 and 8--L. pneumophila serogroup 4 (strain Los Angeles-1)
Lanes 3 and 6--L. pneumophila serogroup 1 (strain Philadelphia-1)
Lanes 4 and 9--L. pneumophila serogroup 5 (strain U8W).

It is pointed out that the affinity purified antibodies for Lanes 1-4 were affinity purified on a column to which carbohydrate antigen from L. pneumophila serogroup 5 (strain U8W) was attached while those for Lanes 6-9 were affinity purified in the same manner on a column having attached carbohydrate antigen of L. pneumophila serogroup 5 (strain Dallas IE).

Figure 2 clearly demonstrates that affinity purified antibodies as herein disclosed of L. pneumophila serogroup 5 react with antigens of L. pneumophila serogroups 1, 2, and 4 in addition to those of serogroup 5.

An ICT assay as described above in which affinity purified antibodies from L. pneumophila serogroup 5 are substituted for affinity purified antibodies from L. pneumophila serogroup 1 is contemplated.

Those skilled in the art of immunochemistry generally, and especially those skilled in immunoassays, will recognize that other materials and ingredients and at times, other procedural steps, can readily be substituted for those specifically recommended herein. A vast array of literature, both patent and non-patent, discusses the design and use of reliable, one-time-use, disposable immunoassay test devices that could be substituted for the preferred ICT device described and recommended herein. It is not intended that the present invention should be limited with respect to substitutable assay devices, materials, ingredients or process steps except insofar as the following claims may so limit it.

## Claims

1. A method of assaying for the presence of a Legionella bacterium or its carbohydrate antigenic component in a fluid, which method comprises the steps of:
(a) harvesting bacterial cells from a culture of a known species, or serogroup of a species, of Legionella bacteria in the form of a wet cell pellet,
(b) suspending the wet cell pellet at are gram per 20 mL in an aqueous alkaline solution, said aqueous alkaline solution comprising 0.1 M aqueous sodium hydroxide, and mixing for 45 minutes, followed by adding strong acid to adjust the pH to acid pH and centrifuging,
(c) separating the supernatant from step (b), adjusting its pH to neutrality and adding thereto a broad spectrum protease enzyme preparation,
(d) digesting the broad spectrum protease enzyme -- neutralized supernatant mixture from step (c) to destroy residual proteins,
(e) adding to the digested mixture from step (d) a weakly alkaline aqueous solution to adjust the pH to the alkaline side, separating out a protein-free carbohydrate antigen on a size exclusion column equilibrated with a weakly alkaline solution and pooling material eluted in the first peak and adjusting its pH to neutrality,
(f) recovering protein-free carbohydrate antigen from the neutral aqueous solution of step (e) and coupling this antigen to a spacer molecule to form a conjugate;
(g) coupling the conjugate from step (f) to a chromatographic affinity column,
(h) purifying raw antibodies to the same known species or serogroup of a species of Legionella bacteria used in step (a) by passing them over the chromatographic affinity column from step (g) having coupled thereto the conjugate of spacer molecule and protein-free carbohydrate antigen from step (f), thus producing purified antigen-specific antibodies, and;
(i) using the purified antibodies from step (h) to detect the presence or absence of an antigenic binding partner for these antibodies in a fluid test sample suspected of containing it.

2. The method of claim 1 in which the bacterium is L. pneumophila serogroup 1 and the protein-free O-carbohydrate antigen recovered in step (f) is the protein-free O-polysaccharide antigen of L. pneumophila serogroup 1.

3. The method of claim 1 in which the bacterium is L. pneumophila serogroup 5 and the protein -free antigen recovered in step (f) is the O-carbohydrate antigen of L. pneumophila serogroup 5.

4. A protein-free carbohydrate antigen obtained from a bacterium which is a species, or serogroup of a species of Legionella pneumophila according to the method of steps (a) to (e) inclusive of claim 1.

5. A protein-free carbohydrate antigen according to claim 4 which is an O-polysaccharide antigen obtained from L. pneumophila serogroup 1.

6. A protein-free carbohydrate antigen according to claim 4 which is an O-carbohydrate antigen obtained from pneumophila serogroup 5.

7. A method according to claim 1 in which in step (b), the adjusted acid pH is 3.0.

8. A method according to claim 1 in which, in step (e) the pH is adjusted to a pH between 10 and 11.

9. A method according to claim 1 in which, after step (e), a lyophilization step is performed.

10. A method for the purification of raw antibodies to a species, or serogroup of species, of Legionella bacteria, which comprises the steps of:
(a) separating from the same species, or serogroup of a species, of Legionella bacteria a protein-free carbohydrate antigen using the method steps (a) to (f) inclusive of claim 1,
(b) coupling the conjugate to an activated chromatographic column;
(c) subjecting said raw antibodies to affinity chromatography on the column from step (c) to obtain purified antigen-specific antibodies, and
(d) eluting from said column the purified antigen-specific antibodies.

11. A method according to claim 10 in which the raw antibodies are antibodies to L. pneumophila serogroup 1 and the protein-free carbohydrate antigen is the protein-free O-polysaccharide antigen of claim 5.

12. A method according to claim 10 in which the raw antibodies are antibodies to L. pneumophila serogroup 5 and the protein-free carbohydrate antigen is the protein-free carbohydrate antigen of claim 6.

13. The purified antigen-specific antibodies to L. pneumophila serogroup 1 and its O-polysaccharide antigen, which are produced by the method of claim 11.

14. The purified antigen-specific antibodies to L. pneumophila serogroup 5 and its carbohydrate antigen, which are produced by the method of claim 12.

15. Purified antibodies to a bacterium of at least one species, or serogroup of a species, of L. pneumophila obtained by the method of claim 10, which purified antibodies exhibit specificity to the carbohydrate antigen of that species, or serogroup of a species.

16. The method of claim 1 in which the spacer molecule of step (f) is a protein molecule.

17. The method of claim 1 in which the known Legionella bacteria are bacteria from one of the serogroups of Legionella pneumophila.

18. The method of claim 17 in which the known Legionella bacteria are bacteria of L. pneumophila serogroup 1, the antigen obtained in step (e) is its protein-free 0-polysaccharide antigen and the purified antibodies to L. pneumophila serogroup 1 bacteria obtained in step (h) are used in step (i) to assay for the presence or absence of L. pneumophila serogroup1 bacterium or its O-polysaccharide antigen.

19. The method of claim 18 wherein the fluid of step (i) is water.

20. The method of claim 18 wherein the fluid of step (i) is a natural fluid of mammalian origin.

21. The method of claim 20 wherein the fluid is human urine.

22. The method of claim 21 in which the fluid is obtained from a patient exhibiting clinical signs of a pneumonia-type illness.

23. The method of claim 22 in which step (i) is an immuno-assay process.

24. The method of claim 23 in which step (i) is an immuno-chromatographic ("ICT") assay process.

25. The method of claim 23 wherein a portion of the purified antigen specific antibodies from step (h) are conjugated to a labeling agent known to display a visible color when said antibodies react with the corresponding antigen.

26. The method of claim 25 wherein the labeling agent is colloidal gold.

27. The method of claim 17 in which the known Legionella bacteria are the bacteria of L. pneumophila serogroup 5, the antigen obtained in step (e) is its protein-free carbohydrate antigen and the purified antibodies obtained from step (h) are capable of detecting in fluids L. pneumophila bacteria from serogroups 1,2, 4 and 5 or their antigenic components.

28. The method of claim 27 in which the purified polyvalent antibodies to L. pneumophila serogroup 5 are utilized to assay for the presence of any or all of L. pneumophila serogroups 1,2, 4 and 5 bacteria or their antigens in a fluid.

29. The method of claim 28 in which the fluid is water.

30. The method of claim 29 in which step (i) is an immuno-assay procedure.

31. The method of claim 30 in which step (i) is an immuno-chromatographic ("ICT") assay process.

32. The method of claim 31 wherein a portion of the purified antigen specific antibodies from step (h) are conjugated to a labeling agent known to display a visible color when said antibodies react with the corresponding antigen.

33. The method of claim 32 wherein the labeling agent is colloidal gold.

34. An ICT assay for the detection of at least one species or serogroup of a species of Legionella bacteria, or a carbohydrate antigen of said bacteria, which comprises
(a) contacting a sample of a fluid suspected of containing said bacteria or its carbohydrate antigen component with an ICT device comprising a strip of bibulous material, which strip has
(i) a zone in which has been embedded a conjugate of
(1) a labeling agent that displays a visible color change upon reaction of antibodies with their corresponding antigenic binding partner and
(2) purified antigen-specific antibodies to the Legionella species, or serogroup of a species, sought to be detected, said antibodies having been purified by passage over a chromatographic affinity column to which is conjugated via a spacer molecule a protein-free carbohydrate antigen of the Legionella species, or serogroup of a species, sought to be detected, which carbohydrate antigen was obtained from a culture of the same Legionella species, or serogroup of a species by conducting the method steps (a) to (e) inclusive of claim 1.
(ii) a second zone having bound thereto the same purified antigen-specific antibodies in unconjugated form, which zone is equipped with a window for viewing color changes,
(b) allowing said sample to flow laterally along said test strip to said first zone,
(c) allowing said sample, together with said conjugate of affinity purified antibodies and label, to flow laterally along said test strip to said second zone, and
(d) within 15 minutes from the commencement of step (a) observing through said window whether a line of color has appeared in said second zone, thereby indicating the presence in the sample of the Legionella bacteria species, or serogroup of a species, that is sought to be detected or the carbohydrate antigen of said species or serogroup of species.

35. The method of claim 34 wherein the fluid suspected of containing at least one species, or serogroup of species, of Legionella bacteria is water.

36. The method of claim 34 wherein the fluid suspected of containing at least one species, or serogroup of a species, of Legionella bacteria is a natural fluid of mammalian origin.

37. The method of claim 36 in which the natural fluid of mammalian origin is human urine.

38. The method of claim 37 in which the labeling agent that displays a visible color change upon reaction of antibodies with their corresponding antigenic binding partner is finely divided metal.

39. The method of claim 38 wherein the labeling agent is colloidal gold.

40. The method of claim 36 wherein the bacteria or antigen thereof to be detected is L. pneumophila serogroup 1 or its O-polysaccharide antigen, the purified antigen-specific antibodies are antibodies to L. pneumophila serogroup 1 and the protein-free antigen conjugated to the chromatographic column used in purification of the antibodies is the protein-free O-polysaccharide antigen 1 of L. pneumophila group obtained from a culture of L. pneumophila serogroup 1 by conducting steps (a) to (e) of claim 1.

41. The method of claim 36 wherein the purified antigen-specific antibodies are antibodies to L. pneumophila serogroup 5, and the protein-free antigen conjugated to the chromatographic column used in purification of the antibodies is a protein-free carbohydrate antigen of L. pneumophila serogroup 5 obtained from a culture of L. pneumophila serogroup 5 by conducting method steps (a) to (e) of claim 1.

## Patentansprüche

1. Verfahren zum Testen auf die Anwesenheit eines Legionella-Bakteriums oder dessen antigenen Kohlenhydratbestandteils in einem Fluid, welches die Schritte umfasst:
(a) Ernten von Bakterien-Zellen von einer Kultur einer bekannten Spezies, oder Serogruppe einer Spezies von Legionella-Bakterien in der Form eines nassen Zell-Pellets;
(b) Suspendieren von einem Gramm des nassen Zell-Pellets pro 20 ml in einer wässrigen alkalischen Lösung, wobei die wässrige alkalische Lösung 0,1 M wässriges Natriumhydroxid umfasst, und Mischen für 45 Minuten, gefolgt von Zugabe einer starken Säure, um den pH-Wert auf einen sauren pH einzustellen und Zentrifugieren,
(c) Abtrennen des Überstandes von Schritt (b), Einstellen dessen pH-Werts auf Neutralität und wobei dazu eine Enzym-Protease-Präparation mit breitem Spektrum zugegeben wird,
(d) Verdauen der Enzym-Protease mit breitem Spektrum -- neutralisierten Überstandsgemischs von Schritt (c), um verbleibende Proteine zu zerstören,
(e) Zugeben einer schwach alkalischen wässrigen Lösung zu dem verdauten Gemisch von Schritt (d), um den pH-Wert auf die alkalische Seite einzustellen, Abtrennen eines proteinfreien Kohlenhydrat-Antigens auf einer Größenausschlusssäule, die mit einer schwach alkalischen Lösung äquilibriert wurde und Vereinigen von Material, das in der ersten Spitze eluiert und Einstellen dessen pH-Werts auf Neutralität,
(f) Rückgewinnen des proteinfreien Kohlenhydrat-Antigens von der neutralen wässrigen Lösung von Schritt (e) und Koppeln dieses Antigens an ein Spacermolekül, um ein Konjugat zu bilden,
(g) Koppeln des Konjugats von Schritt (f) an eine chromatographische Affinitätssäule,
(h) Reinigen roher Antikörper gegen die in Schritt (a) verwendete gleiche bekannte Spezies oder Serogruppe einer Spezies von Legionella-Bakterien, indem sie über die chromatographische Affinitätssäule von Schritt (g) mit dem daran gekoppelten Konjugat von Spacermolekül und proteinfreiem Kohlenhydratantigen von Schritt (f) gegeben werden, wodurch gereinigte antigen-spezifische Antikörper erzeugt werden, und
(i) Verwenden der gereinigten Antikörper von Schritt (h), um die Anwesenheit oder Abwesenheit eines antigenen Bindungspartners für diese Antikörper in einer fluiden Testprobe nachzuweisen, die im Verdacht steht diesen zu enthalten.

2. Verfahren nach Anspruch 1, wobei das Bakterium L. pneumophila der Serogruppe 1 ist und das in Schritt (f) zurückgewonnene proteinfreie O-Kohlenhydrat-Antigen das proteinfreie O-Polysaccharid-Antigen von L. pneumophila der Serogruppe 1 ist.

3. Verfahren nach Anspruch 1, wobei das Bakterium L. pneumophila der Serogruppe 5 ist und das in Schritt (f) zurückgewonnene proteinfreie Antigen das O-Kohlenhydrat-Antigen von L. pneumophila der Serogruppe 5 ist.

4. Proteinfreies Kohlenhydrat-Antigen erhalten von einem Bakterium, das eine Spezies, oder Serogruppe einer Spezies von Legionella pneumophila gemäß einschließlich der Verfahrensschritte (a) bis (e) von Anspruch 1 ist.

5. Proteinfreies Kohlenhydrat-Antigen nach Anspruch 4, das ein von L. pneumophila der Serogruppe 1 erhaltenes O-Polysaccharid-Antigen ist.

6. Proteinfreies Kohlehydrat-Antigen nach Anspruch 4, das ein von Pneumophila der Serogruppe 5 erhaltenes O-Kohlenhydrat-Antigen ist.

7. Verfahren nach Anspruch 1, wobei in Schritt (b) der eingestellte pH-Wert 3,0 beträgt.

8. Verfahren nach Anspruch 1, wobei in Schritt (e) der pH-Wert auf einen pH-Wert zwischen 10 und 11 eingestellt wird.

9. Verfahren nach Anspruch 1, wobei nach Schritt (e) ein Gefriertrocknungsschritt ausgeführt wird.

10. Verfahren zur Reinigung roher Antikörper gegen eine Spezies, oder Serogruppe von Spezies von Legionella-Bakterien, welches die Schritte umfasst von:
(a) Abtrennen eines proteinfreien Kohlenhydrat-Antigens unter Verwendung einschließlich der Verfahrensschritte (a) bis (f) von Anspruch 1 von der gleichen Spezies, oder Serogruppe einer Spezies von Legionella-Bakterien,
(b) Koppeln des Konjugats an eine aktivierte Chromatographiesäule;
(c) Unterziehen der rohen Antikörper einer Affinitätschromatographie auf der Säule von Schritt (c), um gereinigte antigen-spezifische Antikörper zu erhalten, und
(d) Eluieren der gereinigten antigen-spezifischen Antikörper von der Säule.

11. Verfahren nach Anspruch 10, wobei die rohen Antikörper gegen L. pneumophila der Serogruppe 1 gerichtete Antikörper sind und das proteinfreie Kohlenhydrat-Antigen das proteinfreie O-Polysaccharid-Antigen von Anspruch 5 ist.

12. Verfahren nach Anspruch 10, wobei die rohen Antikörper gegen L. pneumophila der Serogruppe 5 gerichtete Antikörper sind und das proteinfreie Kohlenhydrat-Antigen das proteinfreie Kohlenhydrat-Antigen von Anspruch 6 ist.

13. Gereinigte antigen-spezifische Antikörper gegen L. pneumophila der Serogruppe 1 und deren O-Polysaccharid-Antigen, die durch das Verfahren von Anspruch 11 hergestellt werden.

14. Gereinigte antigen-spezifische Antikörper gegen L. pneumophila der Serogruppe 5 und deren Kohlenhydrat-Antigen, die durch das Verfahren von Anspruch 12 hergestellt werden.

15. Gereinigte Antikörper gegen ein Bakterium von mindestens einer Spezies, oder Serogruppe einer Spezies von L. pneumophila, die durch das Verfahren von Anspruch 10 erhalten werden, welche gereinigten Antikörper Spezifität gegen das Kohlenhydrat-Antigen von derjenigen Spezies, oder Serogruppe einer Spezies zeigen.

16. Verfahren nach Anspruch 1, wobei das Spacermolekül von Schritt (f) ein Proteinmolekül ist.

17. Verfahren nach Anspruch 1, wobei die bekannten Legionella-Bakterien Bakterien von einer der Serogruppen von Legionella pneumophila sind.

18. Verfahren nach Anspruch 17, wobei die bekannten Legionella-Bakterien Bakterien von L. pneumophila der Serogruppe 1 sind, das in Schritt (e) erhaltene Antigen deren proteinfreies O-Polysaccharid-Antigen ist und wobei die in Schritt (h) erhaltenen gereinigten Antikörper gegen L. pneumophila der Serogruppe 1 Bakterien in Schritt (i) verwendet werden, um auf die Anwesenheit oder Abwesenheit von einem L. pneumophila der Serogruppe 1 Bakterium oder dessen O-Polysaccharid-Antigen zu testen.

19. Verfahren nach Anspruch 18, wobei das Fluid von Schritt (i) Wasser ist.

20. Verfahren nach Anspruch 18, wobei das Fluid von Schritt (i) ein natürliches Fluid von Säugetierursprung ist.

21. Verfahren nach Anspruch 20, wobei das Fluid menschlicher Urin ist.

22. Verfahren nach Anspruch 21, wobei das Fluid von einem Patienten erhalten wird, der Klinische Anzeichen einer Erkrankung vom Pneumonie-Typ zeigt.

23. Verfahren nach Anspruch 22, wobei Schritt (i) ein Immunoassay-Verfahren ist.

24. Verfahren nach Anspruch 23, wobei Schritt (i) ein Immun-chromatographisches ("ICT") Test-Verfahren ist.

25. Verfahren nach Anspruch 23, wobei ein Anteil der gereinigten antigen-spezifischen Antikörper von Schritt (h) an ein Markierungsmittel konjugiert wird, das bekanntermaßen eine sichtbare Farbe zeigt, wenn die Antikörper mit dem entsprechenden Antigen reagieren.

26. Verfahren nach Anspruch 25, wobei das Markierungsmittel kolloidales Gold ist.

27. Verfahren nach Anspruch 17, wobei die bekannten Legionella-Bakterien die Bakterien von L. pneumophila der Serogruppe 5 sind, das in Schritt (e) erhaltene Antigen deren proteinfreie Kohlenhydrat-Antigen ist und wobei die in Schritt (h) erhaltenen gereinigten Antikörper ermöglichen L. pneumophila Bakterien von Serogruppen 1, 2, 4 und 5 oder deren antigenen Bestandteile in Fluiden nachzuweisen.

28. Verfahren nach Anspruch 27, wobei die gereinigten polyvalenten Antikörper gegen L. pneumophila der Serogruppe 5 dazu verwendet werden, um in einem Fluid auf die Anwesenheit einer beliebigen oder allen von L. pneumophila der Serogruppen 1, 2, 4 und 5 Bakterien oder deren Antigene zu testen.

29. Verfahren nach Anspruch 28, wobei das Fluid Wasser ist.

30. Verfahren nach Anspruch 29, wobei Schritt (i) ein Immunoassay-Verfahren ist.

31. Verfahren nach Anspruch 30, wobei Schritt (i) ein Immun-chromatographisches ("ICT") Test-Verfahren ist.

32. Verfahren nach Anspruch 31, wobei ein Anteil der in Schritt (h) gereinigten antigen-spezifischen Antikörper an ein Markierungsmittel konjugiert wird, das bekanntermaßen eine sichtbare Farbe zeigt, wenn die Antikörper mit dem entsprechenden Antigen reagieren.

33. Verfahren nach Anspruch 32, wobei das Markierungsmittel kolloidales Gold ist.

34. ICT-Assay für den Nachweis von mindestens einer Spezies oder Serogruppe einer Spezies von Legionella-Bakterien, oder einem Kohlenhydrat-Antigen der Bakterien, welcher umfasst:
(a) Inkontaktbringen einer Probe von einem Fluid, die im Verdacht steht die Bakterien oder deren Kohlenhydrat-Antigen-Bestandteil zu enthalten, mit einer ICT-Einrichtung, die einen Streifen saugfähigen Materials umfasst, welcher Streifen aufweist:
(i) einen Bereich in dem eingebettet vorliegt ein Konjugat von
(1) einem Markierungsmittel, das auf Reaktion von Antikörpern mit deren entsprechendem antigenen Bindungspartner eine sichtbare Farbänderung zeigt und
(2) gereinigten antigen-spezifischen Antikörpern gegen die Legionella-Spezies, oder Serogruppe einer Spezies, die nachgewiesen werden sollen, wobei die Antikörper durch Passage über eine chromatographische Affinitätssäule gereinigt wurden, an die über ein Spacermolekül ein proteinfreies Kohlenhydrat-Antigen der nachzuweisenden Legionella-Spezies, oder Serogruppe einer Spezies konjugiert wird, welches Kohlenhydrat-Antigen von einer Kultur der gleichen Legionella-Spezies oder Serogruppe einer Spezies durch einschließliches Ausführen der Verfahrensschritte (a) bis (e) von Anspruch 1 erhalten wurde;
(ii) einen zweiten Bereich, der daran gebunden die gleichen gereinigten antigen-spezifischen Antikörper in nicht-konjugierter Form aufweist, wobei der Bereich mit einem Fenster zum Ansehen von Farbänderungen ausgestattet ist,
(b) Ermöglichen, dass die Probe in Längsrichtung des Teststreifens zu dem ersten Bereich fließt,
(c) Ermöglichen, dass die Probe zusammen mit dem Konjugat von affinitätsgereinigten Antikörpern und Markierung in Längsrichtung des Teststreifens zu dem zweiten Bereich fließt, und
(d) innerhalb 15 Minuten von dem Beginn von Schritt (a) durch das Fenster zu beobachten, ob eine Farblinie in der zweiten Zone erschien, wodurch in der Probe die Anwesenheit von der Legionella-Bakterienspezies, oder Serogruppe einer Spezies angezeigt wird, die nachgewiesen werden sollen, oder des Kohlenhydrat-Antigens der Spezies oder Serogruppe von Spezies.

35. Verfahren nach Anspruch 34, wobei das Fluid, das im Verdacht steht mindestens eine Spezies oder Serogruppe einer Spezies von Legionella-Bakterien zu enthalten, Wasser ist.

36. Verfahren nach Anspruch 34, wobei das Fluid, das im Verdacht steht mindestens eine Spezies oder Serogruppe einer Spezies von Legionella-Bakterien zu enthalten, ein natürliches Fluid von Säugetierursprung ist.

37. Verfahren nach Anspruch 36, wobei das natürliche Fluid von Säugetierursprung menschlicher Urin ist.

38. Verfahren nach Anspruch 37, wobei das Markierungsmittel fein verteiltes Metall ist, das auf Reaktion von Antikörpern mit deren entsprechendem antigenen Bindungspartner eine sichtbare Farbänderung anzeigt.

39. Verfahren nach Anspruch 38, wobei das Markierungsmittel kolloidales Gold ist.

40. Verfahren nach Anspruch 36, wobei die nachzuweisenden Bakterien oder das nachzuweisende Antigen davon L. pneumophila der Serogruppe 1 oder deren O-Polysaccharid-Antigen ist, die gereinigten antigen-spezifischen Antikörper gegen L. pneumophila der Serogruppe 1 gerichtete Antikörper sind und wobei das zur Reinigung der Antikörper verwendete proteinfreie Antigen, das an der chromatographischen Säule konjugiert vorliegt, das proteinfreie O-Polysaccharid-Antigen 1 von L. pneumophila Gruppe ist, das von einer Kultur von L. pneumophila der Serogruppe 1 durch Ausführen der Schritte (a) bis (e) von Anspruch 1 erhalten wird.

41. Verfahren nach Anspruch 36, wobei die gereinigten antigen-spezifischen Antikörper gegen L. pneumophila der Serogruppe 5 gerichtete Antikörper sind, und wobei das zur Reinigung der Antikörper verwendete proteinfreie Antigen, das an der chromatographischen Säule konjugiert vorliegt, ein proteinfreies Kohlenhydrat-Antigen von L. pneumophila der Serogruppe 5 ist, das von einer Kultur von L. pneumophila der Serogruppe 5 durch Ausführen der Verfahrensschritte (a) bis (e) von Anspruch 1 erhalten wird.

## Revendications

1. Une méthode pour tester la présence d'une bactérie Legionella ou de son composant hydrate de carbone antigénique, méthode qui comprend les étapes de :
(a) récolter des cellules de bactéries d'une culture d'une espèce connue, ou d'un sérogroupe d'une espèce, de bactéries Legionella sous la forme d'une boulette cellulaire humide,
(b) suspendre la boulette cellulaire humide à raison de un gramme pour 20 ml dans une solution alcaline aqueuse, ladite solution alcaline aqueuse comprenant 0.1 M d'hydroxyde de sodium aqueux, et mélanger durant 45 minutes, suivi de l'addition d'acide fort pour ajuster le pH à un pH acide et centrifuger ;
(c) séparer le surnageant de l'étape (b), ajuster son pH à neutralité et y ajouter une préparation d'enzyme protéase à large spectre,
(d) digérer le mélange enzyme protéase à large spectre - surnageant neutralisé de l'étape (c) pour détruire les protéines résiduelles,
(e) ajouter au mélange digéré de l'étape (d) une solution aqueuse faiblement alcaline pour ajuster le pH du côté alcalin, séparer un hydrate de carbone antigène exempt de protéine sur une colonne d'exclusion par la taille équilibrée avec une solution faiblement alcaline et rassembler le matériel élué dans le premier pic et ajuster son pH à neutralité,
(f) récupérer l'hydrate de carbone antigène exempt de protéine de la solution aqueuse neutre de l'étape (e) et accrocher cet antigène à une molécule d'espacement pour former un conjugué ;
(g) accrocher le conjugué de l'étape (f) à une colonne chromatographique d'affinité,
(h)purifier les anticorps bruts des mêmes espèces connues ou du sérogroupe d'une espèce de bactéries Legionella utilisées dans l'étape (a) en les passant sur la colonne chromatographique d'affinité de l'étape (g) ayant accroché à elle le conjugué de la molécule d'espacement et de l'hydrate de carbone antigène exempt de protéine de l'étape (f), produire ainsi des anticorps spécifiques d'antigène purifiés, et ;
(i) utiliser les anticorps purifiés de l'étape (h) pour détecter la présence ou l'absence d'un partenaire de liaison antigénique pour ces anticorps dans un échantillon de fluide test suspecté de le contenir.

2. La méthode de la revendication 1 dans laquelle la bactérie est L. pneumophila sérogroupe 1 et le O-hydrate de carbone antigène exempt de protéine récupéré dans l'étape (f) est le O-polysaccharide antigène exempt de protéine de L. pneumophila sérogroupe 1.

3. La méthode de la revendication 1 dans laquelle la bactérie est L. pneumophila sérogroupe 5 et l'antigène exempt de protéine récupéré dans l'étape (f) est le O-hydrate de carbone antigène exempt de protéine de L. pneumophila sérogroupe 5.

4. Un hydrate de carbone antigène exempt de protéine obtenu à partir d'une bactérie qui est une espèce, ou un sérogroupe d'une espèce de Legionella pneumophila selon la méthode des étapes (a) à (e) incluse de la revendication 1.

5. Un hydrate de carbone antigène exempt de protéine selon la revendication 4 qui est un O-hydrate de carbone antigène exempt de protéine obtenu à partir de L. pneumophila sérogroupe 1.

6. Un hydrate de carbone antigène exempt de protéine selon la revendication 4 qui est un O-hydrate de carbone antigène exempt de protéine obtenu à partir de pneumophila sérogroupe 5.

7. Une méthode selon la revendication 1 dans laquelle dans l'étape (b), le pH ajusté acide est 3.0.

8. Une méthode selon la revendication 1 dans laquelle, dans l'étape (e) le pH est ajusté à un pH entre 10 et 11.

9. Une méthode selon la revendication 1 dans laquelle, après l'étape (e), une étape de lyophilisation est appliquée.

10. Une méthode pour la purification d'anticorps bruts d'une espèce, ou d'un sérogroupe d'espèce, de bactéries Legionella, qui comprend les étapes de :
(a) séparer de la même espèce, ou du sérogroupe d'une espèce, de bactéries Legionella un hydrate de carbone antigène exempt de protéine en utilisant la méthode des étapes (a) à (f) incluse de la revendication 1,
(b) accrocher le conjugué à une colonne chromatographique activée ;
(c) soumettre lesdits anticorps bruts à une chromatographie d'affinité sur la colonne de l'étape (c) pour obtenir des anticorps spécifiques d'antigènes purifiés, et
(d) éluer de ladite colonne les anticorps spécifiques d'antigènes purifiés.

11. Une méthode selon la revendication 10 dans laquelle les anticorps bruts sont des anticorps de L. pneumophila sérogroupe 1 et l'hydrate de carbone antigène exempt de protéine est le O-polysaccharide antigène exempt de protéine de la revendication 5.

12. Une méthode selon la revendication 10 dans laquelle les anticorps bruts sont des anticorps de L. pneumophila sérogroupe 5 et l'hydrate de carbone antigène exempt de protéine est l'hydrate de carbone antigène exempt de protéine de la revendication 6.

13. Les anticorps spécifiques d'antigènes purifiés de L. pneumophila sérogroupe 1 et son O-polysaccharide antigène, qui sont produits par la méthode de la revendication 11.

14. Les anticorps spécifiques d'antigènes purifiés de L. pneumophila sérogroupe 5 et son hydrate de carbone antigène, qui sont produits par la méthode de la revendication 12.

15. Anticorps purifiés d'une bactérie d'au moins une espèce, ou sérogroupe d'une espèce, de L. pneumophila obtenus par la méthode de la revendication 10, anticorps purifiés qui présentent une spécificité à l'hydrate de carbone antigène de cette espèce, ou sérogroupe d'une espèce.

16. La méthode de la revendication 1 dans laquelle la molécule d'espacement de l'étape (f) est une molécule de protéine.

17. La méthode de la revendication 1 dans laquelle les bactéries Legionella connues sont des bactéries de l'un des sérogroupes de Legionella pneumophila.

18. La méthode de la revendication 17 dans laquelle les bactéries Legionella connues sont des bactéries de Legionella pneumophila sérogroupe 1, l'antigène obtenu dans l'étape (e) est son O-polysaccharide antigène exempt de protéine et les anticorps purifiés de bactéries L. pneumophila ségroupe 1 obtenus dans l'étape (h) sont utilisés dans l'étape (i) pour tester la présence ou l'absence de bactérie L. pneumophila sérogroupe 1 ou de son O-polysaccharide antigène.

19. La méthode de la revendication 18 dans laquelle le fluide de l'étape (i) est de l'eau.

20. La méthode de la revendication 18 dans laquelle le fluide de l'étape (i) un fluide naturel d'origine mammifère.

21. La méthode de la revendication 20 dans laquelle le fluide est de l'urine humaine.

22. La méthode de la revendication 21 dans laquelle le fluide est obtenu d'un patient présentant des signes cliniques d'une maladie de type pneumonie.

23. La méthode de la revendication 22 dans laquelle l'étape (i) est un procédé de test immunitaire.

24. La méthode de la revendication 23 dans laquelle l'étape (i) est un procédé de test immuno-chromatographique (« ICT »).

25. La méthode de la revendication 23 dans laquelle une portion des anticorps purifiés spécifiques d'antigène de l'étape (h) sont conjugués à un agent de marquage connu pour afficher une couleur visible lorsque lesdits anticorps réagissent avec l'antigène correspondant.

26. La méthode de la revendication 25 dans laquelle l'agent de marquage est de l'or colloïdal.

27. La méthode de la revendication 17 dans laquelle les bactéries Legionella connues sont des bactéries de L. pneumophila sérogroupe 5, l'antigène obtenu dans l'étape (e) est son hydrate de carbone antigène exempt de protéine et les anticorps purifiés obtenus dans l'étape (h) sont capables de détecter dans des fluides des bactéries L. pneumophila des sérogroupes 1, 2, 4 et 5 ou leurs composants antigéniques.

28. La méthode de la revendication 27 dans laquelle les anticorps polyvalents purifiés de L. pneumophila sérogroupe 5 sont utilisés pour tester la présence de l'une ou de toutes les bactéries L. pneumophila des sérogroupes 1, 2, 4 et 5 ou de leurs antigènes dans un fluide.

29. La méthode de la revendication 28 dans laquelle le fluide est de l'eau.

30. La méthode de la revendication 29 dans laquelle l'étape (i) est un procédé de test immunitaire.

31. La méthode de la revendication 30 dans laquelle l'étape (i) est un procédé de test immuno-chromatographique (« ICT »).

32. La méthode de la revendication 31 dans laquelle une portion des anticorps purifiés spécifiques d'antigène de l'étape (h) sont conjugués à un agent de marquage connu pour afficher une couleur visible lorsque lesdits anticorps réagissent avec l'antigène correspondant.

33. La méthode de la revendication 32 dans laquelle l'agent de marquage est de l'or colloïdal.

34. Un test ICT pour la détection d'au moins une espèce ou sérogroupe d'une espèce de bactéries Legionella, ou d'un hydrate de carbone antigène desdites bactéries, qui comprend
a) contacter un échantillon de liquide suspecté de contenir lesdites bactéries ou son composa nt hydrate de carbone antigène avec un appareil ICT comprenant une bande en matière absorbante, bande qui possède
(i) une zone dans laquelle a été imprégné un conjugué de
(1)un agent de marquage qui affiche un changement de couleur visible après réaction des anticorps avec leurs partenaires de liaison antigéniques, et
(2) des anticorps purifiés spécifiques de l'espèce Legionella, ou du sérogroupe d'une espèce, que l'on cherche à détecter, lesdits anticorps ayant été purifiés par passage sur une colonne chromatographique à laquelle est conjugué par une molécule d'espacement un hydrate de carbone antigène exempt de protéine de l'espèce Legionella, ou sérogroupe d'une espèce, que l'on cherche à détecter, hydrate de carbone antigène qui a été obtenu à partir d'une culture de la même espèce de Legionella, ou sérogroupe d'une espèce en appliquant la méthode des étapes (a) à (e) incluse de la revendication 1 ;
(ii) une seconde zone à laquelle ayant liés à elle les mêmes anticorps spécifiques d'antigènes purifiés sous forme non conjuguée, zone qui est équipée d'une fenêtre pour visualiser les changements de couleur,
b) laisser s'écouler latéralement ledit échantillon le long de ladite bande test vers ladite première zone,
c) laisser ledit échantillon, ensemble avec lesdits anticorps purifiés de conjugués d'affinité et le marqueur, s'écouler latéralement le long de ladite bande vers ladite seconde zone, et
d) en 15 min à compter du début de l'étape (a), observer au travers de ladite fenêtre si une ligne de couleur est apparue dans ladite seconde zone, indiquant par ce moyen la présence dans l'échantillon d'espèces de bactéries Legionella, ou de sérogroupe d'une espèce, que l'on cherche à détecter ou de l'hydrate de carbone antigène de ladite espèce ou sérogroupe d'espèces.

35. La méthode de la revendication 34 dans laquelle le fluide suspecté de contenir au moins une espèce, ou sérogroupe d'espèce, de bactéries Legionella est de l'eau.

36. La méthode de la revendication 34 dans laquelle le fluide suspecté de contenir au moins une espèce, ou sérogroupe d'espèce, de bactéries Legionella est un fluide naturel d'origine mammifère.

37. La méthode de la revendication 36 dans laquelle le fluide naturel d'origine mammifère est de l'urine humaine.

38. La méthode de la revendication 37 dans laquelle l'argent de marquage qui affiche un changement de couleur visible après réaction des anticorps avec leur partenaire de liaison antigénique correspondant est du métal finement divisé.

39. La méthode de la revendication 38 dans laquelle l'agent de marquage est de l'or colloïdal.

40. La méthode de la revendication 36 dans laquelle la bactérie ou l'antigène de celle-ci à détecter est L. pneumophila sérogroupe 1 ou son O-polysaccharide antigène, les anticorps spécifiques d'antigène purifiés sont des anticorps de L. pneumophila sérogroupe 1 et l'antigène exempt de protéine conjugué à la colonne chromatographique utilisée dans la purification des anticorps est le O-polysaccharide antigène 1 exempt de protéine du groupe L. pneumophila obtenu à partir d'une culture de L. pneumophila sérogroupe 1 en conduisant les étapes (a) à (e) de la revendication 1.

41. La méthode de la revendication 36 dans laquelle les anticorps spécifiques d'antigène purifiés sont des anticorps de L. pneumophila sérogroupe 5, et l'antigène exempt de protéine conjugué à la colonne chromatographique utilisée dans la purification des anticorps est un hydrate de carbone antigène exempt de protéine de L. pneumophila sérogroupe 5 obtenu à partir d'une culture de L. pneumophila sérogroupe 5 en conduisant la méthode des étapes (a) à (e) de la revendication 1.
